# FASCICULE DE BREVET EUROPEEN

(11) **EP 2 376 012 B1**
(45) Date de publication et mention de la délivrance du brevet: **07.09.2016**
(21) Numéro de dépôt: 09763988.4
(22) Date de dépôt: 30.10.2009
(51) Int. Cl.: A61N 5/10

(54) **DISPOSITIF POUR POSITIONNER UN PATIENT PAR RAPPORT À UN RAYONNEMENT**
VORRICHTUNG ZUR LAGERUNG EINES PATIENTEN RELATIV ZU EINER STRAHLUNG
DEVICE FOR POSITIONING A PATIENT RELATIVE TO A RADIATION

(30) Priorité: 31.10.2008 FR 0857442
(43) Date de publication de la demande: 19.10.2011
(73) Titulaire: Bureau Français d'Ingénierie, 77710 Villebeon (FR); Institut Curie, 75005 Paris (FR)
(72) Inventeur: PINAULT, Samuel, F-78460 Chevreuse (FR); FERRAND, Régis, F-78000 Versailles (FR); CHEMOUNY, Jérôme, F-77270 Villeparisis (FR)
(74) Mandataire: Pontet Allano & Associes
(86) Numéro de dépôt international: PCT/FR2009/052103
(87) Numéro de publication internationale: WO 2010/049660

(56) Documents cités:
- WO-A-2007/112602
- WO-A-2008/005129
- WO-A-2008/110170
- US-A- 6 094 760
- US-A1- 2003 048 875
- US-A1- 2005 234 327
- MEGGIOLARO M A ET AL: "Geometric and elastic error calibration of a high accuracy patient positioning system" MECHANISM AND MACHINE THEORY, PERGAMON, vol. 40, no. 4, 1 avril 2005 (2005-04-01), pages 415-427, XP025259605 ISSN: 0094-114X [extrait le 2005-04-01]

## Description

La présente invention se rapporte à un dispositif de positionnement d'un patient par rapport à un faisceau de radiothérapie externe. Elle trouve une application particulièrement avantageuse dans le domaine de la radiothérapie où l'on utilise des faisceaux d'irradiation pour des traitements nécessitant une très grande précision par exemple pour le mélanome de la choroïde ou pour certaines tumeurs intracrâniennes. Lorsqu'une tumeur est notamment localisée à la base du crâne d'un patient, la radiothérapie a pour objet d'irradier les zones malignes tout en évitant de toucher des organes critiques, comme les nerfs optiques, le tronc cérébral, l'oreille interne ou la moelle épinière qui se trouvent proches. Il est donc primordial de positionner correctement le patient par rapport au rayonnement destiné à irradier une tumeur. D'une façon générale, en radiothérapie, le patient est installé sur une table ou une chaise puis positionné à l'aide d'un appareillage électromécanique.

Toutefois, la présente invention est d'un cadre plus large puisqu'elle peut s'appliquer à d'autres domaines nécessitant de positionner avec précision un patient ou tout autre objet. La présente invention peut par exemple s'appliquer à des applications médicales de tout ordre, nécessitant la mise en place précise et rapide d'un patient par rapport à un référentiel donné.

Dans l'art antérieur, le document US 2005/0234327 décrit un dispositif de positionnement d'un patient comprenant un plateau pour porter le patient, un bras robotique de morphologie « scara » doté d'une liaison prismatique permettant des déplacements selon un axe vertical, de deux liaisons rotoïdes verticales consécutives et d'un poignet robotique à axes concourants. Le défaut d'un tel dispositif est la modélisation exacte de l'élasticité mécanique lorsque le bras robotique est étiré au maximum avec un patient installé dessus. Un système de table de correction logicielle, évolué, doit être mis en place afin d'obtenir les précisions requises pour une application médicale. Par ailleurs, l'axe de translation verticale subit des contraintes considérables, ce qui peut limiter la durée de vie des composants de liaison et en augmenter le dimensionnement. En outre, cet axe de translation nécessite l'installation du dispositif dans une fosse d'une profondeur supérieure à un mètre.

Par ailleurs, le document « Géométrie and elastic error calibration of a high accuracy patient positionning system », Marco A. Meggiolaro et al., Mechanism and Machine Theory 40 (2005) 415-427, 12 September 2004, décrit une méthode de compensation des déformations d'un autre système électromécanique pour le positionnement d'un patient. Le modèle robotique décrit est doté de trois axes linéaires pour les translations verticales, latérales et longitudinales. Il possède en plus trois axes de rotation non concourants sous l'interface de fixation avec la table de traitement. Ce positionneur à six degrés de libertés offre des possibilités limitées de tangage et roulis (± 3 degrés). En outre, la commande de ce dispositif ne permet pas la réalisation de déplacements évolués comme des rotations autour d'un repère virtuel distant du poignet, lié à la tumeur comme le propose tout système robotique. Cet appareil présente les mêmes défauts que le système précédent, à savoir l'élasticité mécanique et l'obligation d'une fosse profonde dans la salle de traitement.

Le document WO2007/017211 décrit un dispositif de positionnement utilisant un robot industriel doté d'une pluralité d'axes de rotation apte à positionner convenablement un patient. Cependant, un tel dispositif est très encombrant et nécessite une fosse de grande dimension apte à accueillir la partie basse du dispositif de positionnement. L'installation d'un tel dispositif dans une salle de traitement est donc très onéreuse et présente de nombreuses contraintes.

La présente invention a pour but de remédier aux inconvénients de l'art antérieur en proposant un nouveau dispositif de positionnement compact et très rigide, possédant intrinsèquement une grande précision et évitant le recours à une fosse profonde.

L'invention a pour objet de développer un robot de positionnement simple d'installation.

Enfin, l'invention a pour but de proposer un contrôle performant du robot de positionnement afin de garantir au mieux la sécurité du patient et du personnel de soins durant les déplacements.

On atteint au moins l'un des buts précités avec un dispositif selon la revendication 1.

L'invention constitue avantageusement un robot de positionnement très compact, mais capable d'évoluer avec une très grande précision dans un espace étendu. L'encombrement est très réduit par rapport aux dispositifs de l'art antérieur. En effet, la géométrie décrite diffère des morphologies habituelles par l'ordre et les angles utilisés pour l'agencement des segments mécaniques constituant le robot. Les concepts « scara », poly articulés ou hexapodes, présentés précédemment, ont l'inconvénient majeur de nécessiter une fosse profonde dans le sol.

En outre, la présente invention est innovante en termes d'encombrement et de compacité en position de repli dans la salle. Pour rendre l'espace de travail d'un tel système, optimal, il doit être centré sur un plan dont l'altitude par rapport au sol peut varier de un mètre vingt à un mètre cinquante. Cette contrainte est inhérente au principe du traitement par faisceau de radiothérapie.

L'objet de la présente invention est d'autant plus remarquable qu'il constitue un robot de positionnement hybride, la liaison entre l'épaule et le coude de la morphologie poly articulée dite « série » (robot à mouvements de type bras humain) est avantageusement remplacée par le couplage de l'axe linéaire au sol et de la pièce de jonction sur pivot vertical précédemment décrite. Ces changements résolvent les deux contraintes de mobilité (axe de translation) et de compacité (pièce de jonction). Le robot est compact parce que le rail permet de déplacer l'ensemble du bras robotique (de la pièce de jonction jusqu'au support du patient) par translation jusqu'à l'emplacement prévu par le planning de traitement. Le bras robotique, avec ses multiples degrés de liberté, peut ensuite effectuer des mouvements à faible débattement mais nécessitant une grande précision pour optimiser la position de la cible à traiter dans le référentiel de traitement.

Ce robot de positionnement compact à six degrés de liberté permet de limiter la profondeur de la fosse et est capable de transporter de fortes charges d'au moins 250kg avec l'encombrement d'une table de radiothérapie standard. Avec un tel rail linéaire selon l'invention, on peut prévoir un bras robotique court, d'environ 1m30. Ce robot de positionnement présente une raideur et une compacité accrue par rapport à un système de l'art antérieur dans la mesure où ce robot ne dispose pas d'axe déporté, loin de la base.

L'invention, de part la compacité de sa morphologie, possède un centre de gravité assez proche de sa base pour limiter l'effet de porte à faux observé sur les dispositifs de l'art antérieur. Tout le poids du robot de positionnement est donc réparti au sol, ce qui améliore la stabilité et la rigidité du dispositif.

Le robot de positionnement selon l'invention permet d'optimiser et d'adapter l'espace de travail à une application donnée. En effet, la taille du rail linéaire peut varier en fonction de l'espace disponible et des fonctionnalités optionnelles désirées. Il est ainsi envisagé d'augmenter la longueur de l'axe afin de réaliser un pré-positionnement du patient grâce à un imageur scanner d'un côté de la salle puis d'envoyer directement le patient en position de traitement sous l'appareil de radiothérapie par une translation pure de la base du robot. Cette fonctionnalité remplacerait avantageusement les procédures actuelles, fastidieuses et peu adaptées pour certains traitements en pédiatrie notamment.

Selon un autre mode d'assemblage des axes du robot, le rail linéaire peut être fixé à une partie pivotante d'une base, l'axe de pivot de cette partie pivotante étant vertical. Dans le cas d'un tel assemblage, on peut installer le système dans une fosse peu profonde (de l'ordre de 300mm maximum de profondeur). Par l'ajout de ce nouvel axe, le robot comporte sept axes combinant six rotations et une translation, pour une mobilité maximale de six degrés de liberté. Cet ajout rend l'architecture redondante et permet d'atteindre des positions difficiles d'accès dans un milieu encombré comme une salle de traitement.

Pratiquement, pour assurer une bonne précision, on prévoit que la partie pivotante pivote par rapport à une partie fixe de la base au moyen d'un roulement à billes. La partie fixe de la base peut comprendre plusieurs plots en profilé aluminium fixés au sol dans une petite fosse par exemple. L'assemblage ainsi réalisé permet de réaliser une rotation de l'ensemble rail linéaire plus bras robotique de 360°. L'enveloppe de travail d'un tel appareillage correspond à une demi-sphère, sans zone morte.

De préférence, l'axe de rotation vertical du rail linéaire par rapport à la base est confondu avec l'axe ou l'aplomb dudit faisceau de rayonnement. Ainsi, lorsqu'une cible, par exemple dans la tête d'un patient, est convenablement positionnée par rapport au rayonnement, il suffit d'une rotation pure de l'axe au sol, pour faire tourner le patient, changer l'angle d'incidence du rayonnement, tout en gardant la cible dans l'axe. Ceci permet d'envisager un traitement dynamique du patient en combinant le déplacement du robot avec la forme du collimateur multi lames de mise en forme du faisceau.

Avantageusement, le bras robotique comprend un avant bras coulissant. L'intérêt est d'avoir un volume de calibration lié au terminal du robot (dans lequel le robot est très précis) qui suit l'axe linéaire. Ce volume se déplace selon un axe très précis (précision axe linéaire <0.1mm) et l'on peut avoir un volume de précision suivant en permanence la zone à traiter. Le terminal, parfois appelé effecteur, correspond à l'extrémité du robot (dispositif de positionnement). Il s'agit généralement d'une plaque d'accroche entre le robot et le support de patient.

Avec un rail linéaire selon l'invention, il est alors possible de simplifier la calibration du dispositif de positionnement. En effet, on peut réaliser un nombre de points de mesure limité, par exemple huit points aux coins d'un volume cubique. On calcule ensuite dans ce volume un certain nombre de positions cartésiennes maximisant des variations articulaires importantes pour chaque déplacement. Le robot aura une précision très élevée dans ce volume, lié à son terminal, qui se déplacera très précisément suivant l'axe linéaire.

Le bras robotique peut être relié à la pièce de jonction de façon pivotante selon un axe de rotation horizontal.

Selon une autre variante de l'invention, le bras robotique est relié à la pièce de jonction de façon pivotante selon un axe de rotation incliné par rapport à l'horizontal d'un angle compris entre 0° et 90°. De préférence, l'axe de rotation du bras robotique par rapport à la pièce de jonction est incliné par rapport à l'horizontal d'un angle compris entre 45 et 60 degrés. Cette inclinaison est notamment utile pour l'inversion du bras (passage du bras de l'avant du robot vers l'arrière) dans un espace confiné avec une hauteur sous plafond réduite.

Par ailleurs, la pièce de jonction peut être reliée de façon pivotante selon un axe vertical à une embase qui est reliée de façon coulissante audit au moins un rail linéaire.

Le poignet est relié au support de patient au moyen d'un changeur d'outil électropneumatique standard.

Selon une caractéristique avantageuse de l'invention, la pièce de jonction se déplace par roulement sur le rail linéaire conçu en profilé acier en « U ».

Avantageusement, le dispositif selon l'invention est conçu de manière à limiter la manutention lors de l'installation dans la salle de traitement, grâce à l'ajout de roues amovibles ou escamotables qui supportent le dispositif lors d'une installation. Ces roues peuvent être fixées sur la base du châssis du dispositif. Ceci facilite la manutention du dispositif et permet un remplacement facile d'un positionneur dans une salle existante.

Selon une caractéristique avantageuse de l'invention, le dispositif comprend un plancher mobile constitué de deux demi-planchers reliés à la pièce de jonction et disposés de part et d'autre de cette pièce de jonction de façon à couvrir constamment le rail linéaire lorsque la pièce de jonction se déplace ; chaque demi-plancher est constitué de plusieurs lames reliées entre elles en chaîne par des articulations, de telle sorte qu'en position de repli d'un demi-plancher, au moins une partie des lames de ce demi-plancher est rangée en accordéon sous un plan horizontal qui contient ce demi-plancher en position dépliée.

De préférence, le plancher coulisse sur deux jeux de rails secondaires :
- un premier jeu de rails secondaires internes sur lesquels coulisse la moitié des articulations, ces deux rails secondaires internes étant parallèles et écartés l'un de l'autre ; ces deux rails secondaires internes présentant une partie linéaire haute, un point de flexion et une partie linéaire basse ;
- un second jeu constitué de deux rails secondaires externes sur lesquels coulisse l'autre moitié des articulations, ces deux rails secondaires externes étant parallèles et écartés d'une distance supérieure à la distance d'écartement des deux rails secondaires internes ; ces deux rails secondaires externes étant linéaires dans un plan horizontal sensiblement à la même hauteur que la partie linéaire haute des rails secondaires internes ;
et pour deux articulations successives, l'une est portée par le premier jeu de rails secondaires, l'autre étant portée par le second jeu de rails secondaires.

Avantageusement, chaque demi-plancher présente en position dépliée une partie plate proche de la pièce de jonction, et en position de repli une partie repliée en accordéon entre la partie linéaire basse des rails secondaires internes et les rails secondaires externes.

Selon une variante avantageuse de l'invention, le plancher est fixe. Dans ce cas, le rail linéaire est constitué par une pluralité d'éléments modulaires fixés au sol et connectés entre eux. Ces éléments modulaires peuvent présenter par exemple une longueur de 1m et permettent d'adapter la course du robot à une application donnée telle que la disposition d'un scanner d'un côté de la salle et d'un accélérateur de traitement d'un autre côté.

Selon un autre aspect de l'invention, il est proposé un système de positionnement comprenant un dispositif de positionnement tel que défini précédemment, une unité de traitement intégrant un logiciel de supervision, une série de capteurs de recalage du patient et notamment une série de capteurs de sécurité.

La vérification des déplacements est assurée par la mise en place de capteurs et logiciel. Les capteurs utilisés sont pour partie des capteurs évolués tels qu'un capteur stéréovision infrarouge capable de suivre le déplacement de cibles à géométrie connue, des capteurs rayons X, des caméras industrielles et un capteur d'effort sur l'organe terminal du robot. L'ensemble des informations recueillies par ces capteurs convergent vers un même centre de traitement, appelé superviseur. Le superviseur connaît l'état courant de l'environnement du robot et peut le comparer avec la configuration théorique de la salle pour la tâche demandée. Cette comparaison s'accompagne d'une modification éventuelle de la trajectoire en cas d'évitement d'obstacles, d'une diminution de la vitesse à l'approche d'une zone potentiellement dangereuse ou d'un avertissement à l'opérateur en cas de problème. Chaque changement par rapport au scénario original étant signalé à l'opérateur dans l'interface de supervision. La sécurité est renforcée par l'ajout d'une couche de capteurs bas niveau : résolveurs, accéléromètres, compteurs impulsionnels sur chaque axe, inclinomètres, bandes anti collisions, ayant pour fonction de couper la puissance du robot en cas de dépassement des limites imposées par les normes régissant l'installation des systèmes électromécaniques en milieu médical.
Selon l'invention, l'unité de traitement peut comprendre un module de visualisation 3D doté :
- d'une modélisation 3D virtuelle de la salle de traitement où se situe le dispositif de positionnement ;
- d'une modélisation 3D virtuelle du dispositif de positionnement ; cette modélisation étant paramétrée pour représenter le positionnement temps réel du dispositif de positionnement à partir de capteurs, et pour définir une enveloppe virtuelle autour au moins d'un élément du dispositif de positionnement de façon à générer un signal d'alerte lorsque l'enveloppe virtuelle entre en collision avec une représentation virtuelle d'un élément de la salle de traitement.

L'unité de traitement peut être paramétrée de façon à déclencher, en réponse au signal d'alerte, un processus anticollision consistant à arrêter le dispositif de positionnement ou à réorienter le mouvement du dispositif de positionnement.

Avec le système selon l'invention, une commande déportée du robot de positionnement améliore la sécurité des déplacements grâce à l'optimisation des trajectoires choisies et à l'interprétation facilitée des ordres à envoyer. Le module de visualisation 3D est notamment utile pour le rendu visuel de la scène et de l'évolution du robot. A partir des mesures des capteurs (position du robot) et de la simulation précise de chaque partie du système (accessoire de traitement, table ou chaise), on peut anticiper et éviter les collisions. Cette fonctionnalité est avantageusement basée sur des enveloppes virtuelles autour de chacun des éléments (fixes ou mobiles) présents dans la salle de traitement. Ce mode de réalisation constitue un système anticollision permettant le déplacement du robot dans la salle de traitement sans risque de collision.

Selon une autre caractéristique avantageuse, le dispositif selon l'invention comprend au moins un capteur d'effort fixé au poignet, de préférence au terminal du dispositif de positionnement, et relié à une unité de traitement contrôlant ledit dispositif de positionnement de façon à réaliser une co-manipulation en accompagnant tout effort détecté par ledit au moins un capteur d'effort. Plus précisément, le capteur d'effort peut comprendre six jauges de contraintes. Les efforts détectés sont transmis à l'unité de traitement (un ordinateur par exemple) qui les traite et renvoie au robot la commande de déplacement dans le sens de l'effort. Cette boucle de commande permet à un utilisateur de manipuler l'outil sans aucune contrainte de masse. Les inerties, et le poids sont compensés par le robot. Les possibilités de mouvements sont celles du robot, c'est-à-dire les six degrés de liberté, trois translations et trois rotations. Ce mode de réalisation permet une manipulation manuelle du robot. L'objectif est notamment de déplacer un patient posé sur le support en co-manipulation pour l'aligner intuitivement devant un appareil d'imagerie ou de traitement à l'aide de systèmes lasers standards. Cette opération de placement à la main du patient permet de diminuer les temps de mise en position. Il s'agit d'un pré-positionnement qui peut aussi être avantageusement utilisé pour une extraction d'urgence intuitive du patient en cas de malaise. Cette méthode est intuitive car l'effort qu'un utilisateur exerce sur le robot est relayé par un déplacement sous asservissement du dispositif de positionnement qui commande un système électromécanique.

Le capteur d'effort peut également être utilisé comme mesure de charge embarquée. Cette mesure est utilisée pour régler les paramètres d'asservissement à la co-manipulation, mais également pour avoir une idée des déformations subies par le robot et ainsi les compenser. Cette mesure directe du capteur est automatisée et par conséquence complètement transparente pour l'utilisateur.

Le capteur d'effort peut encore être utilisé pour la détection de collisions avec des objets mobiles. Lors d'un déplacement du robot suivant une trajectoire prédéfinie ou dynamique, ou pour toute autre manipulation, l'outil peut rentrer en collision avec d'autres éléments de l'environnement (humain, chariot, ...). Ces collisions engendrent des efforts inattendus sur le capteur, on peut donc mettre le robot en arrêt d'urgence pour éviter d'endommager l'une ou l'autre des parties en collision.

D'autres avantages et caractéristiques de l'invention apparaîtront à l'examen de la description détaillée d'un mode de mise en oeuvre nullement limitatif, et des dessins annexés, sur lesquels :
Figure 1 est une vue schématique illustrant le principe cinématique d'un robot de positionnement selon l'invention,
Figure 2 est une première vue schématique latérale du dispositif de positionnement selon l'invention,
Figure 3 est une seconde vue schématique latérale du dispositif de positionnement selon l'invention,
Figure 4 est une vue schématique illustrant un espace de travail dans lequel le robot de positionnement selon l'invention peut se mouvoir,
Figure 5 est une vue schématique d'un robot de positionnement selon l'invention pour lequel le rail linéaire est fixé au sol,
Figure 6 est une vue schématique du robot de positionnement selon l'invention dans une position déployée,
Figures 7 et 8 sont des vues schématiques illustrant un mode de réalisation dans lequel le plancher sur le rail linéaire est mobile,
Figure 9 est une vue schématique d'un mode de réalisation où le plancher est fixe et composé d'éléments modulaires ;
Figure 10 est une vue schématique de côté du robot de positionnement de la figure 9 sans le support de patient ; et
Figure 11 est une représentation virtuelle d'un support de patient du robot de positionnement de la figure 9.

Un robot de positionnement selon l'invention est généralement destiné à positionner un patient par rapport à un rayonnement ionisant lors d'une radiothérapie externe. Un tel robot de positionnement est disposé dans une salle dimensionnée pour de tels traitements thérapeutiques. Cette salle est équipée d'un accélérateur de particules qui est apte à générer un rayonnement focalisé sur la tumeur à traiter dans le corps du patient. On comprendra aisément que le positionnement du patient doit être le plus précis possible et stable tout au long du traitement.

Un robot de positionnement est un bras articulé qui porte une table support ou une chaise support ou tout autre moyen de support sur lequel un patient se place. Les articulations du robot de positionnement sont commandées par une unité de traitement et sont aptes à positionner la tumeur du patient en tout point à l'intérieur d'un espace de travail tridimensionnel.

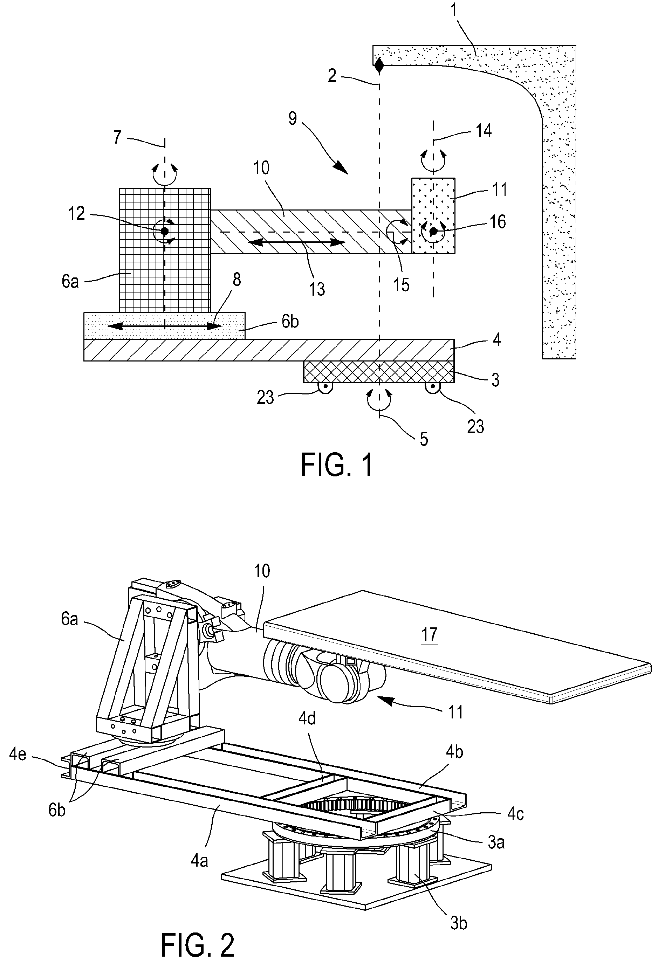

Sur la figure 1 on voit une vue schématique illustrant le principe cinématique d'un robot de positionnement selon l'invention. Un générateur de particules 1 est prévu pour générer un rayonnement selon un axe vertical 2 vers le bas.

Le robot de positionnement comporte une base 3 sur laquelle peut pivoter un rail linéaire 4 selon un axe de rotation vertical 5. Avantageusement, cet axe de rotation vertical 5 peut être confondu avec l'axe 2 du rayonnement. Le robot de positionnement comporte également une pièce de jonction 6a fixée sur une embase 6b de façon pivotante selon un axe de rotation 7 vertical. L'embase 6b peut se déplacer linéairement, par translation, sur le rail linéaire 4 selon un axe de translation 8 horizontal. La pièce de jonction 6a porte un bras robotique 9 composé d'un avant bras 10 et d'un poignet 11. L'avant bras 10 est fixé à la pièce de jonction 6a de façon pivotante selon un axe de rotation 12 horizontal. De préférence, l'avant bras 10 est de type coulissant de sorte qu'il peut s'allonger linéairement selon un axe linéaire 13. L'extrémité de l'avant bras 10 la plus éloignée de la pièce de jonction 6a porte le poignet 11 à trois degrés de liberté selon trois axes de rotation orthogonaux 14, 15 et 16 et concourants. Ce poignet 11 est fixé sur la partie coulissante de l'avant bras 10. La table support (non représentée), destinée à accueillir le patient, se fixe généralement à l'horizontal sur le poignet 11.

De façon conventionnelle, le robot de positionnement selon l'invention est relié à une unité de traitement (non représentée) apte à gérer les articulations de ce robot de positionnement afin de placer la tumeur d'un patient à une position prédéterminée. Cette unité de traitement récupère en temps réel les positions articulaires de chacun des axes grâce à des encodeurs fixés aux moteurs. Ensuite, à partir des paramètres géométriques théoriques issus de la calibration du robot, l'unité de traitement est en mesure de fournir à l'utilisateur une position cartésienne du repère de la tumeur par rapport au repère virtuel de traitement. Ainsi, l'utilisateur peut aisément interpréter cette position et vérifier qu'elle est conforme à la prescription. De la même manière, l'utilisateur peut valider une correction à appliquer au repère de la tumeur dans le repère de traitement conformément à la proposition du système utilisé pour le recalage du patient (scanner, rayons X, infrarouge). Ensuite, cet ordre est analysé et retranscrit en commande articulaire au robot.

L'invention se voit de plus doté d'un logiciel de visualisation 3D avancé, basé sur un modèle théorique de la scène et sur les informations récupérées par les différents capteurs en présence. Cette modélisation fine de la procédure de positionnement permet d'envisager à terme un déport des commandes en dehors de la salle de traitement. Ce déport permettra avantageusement de déplacer le positionneur à distance et ainsi de réduire le temps perdu, par l'opérateur, à rentrer dans la salle pour changer l'incidence de traitement du patient.

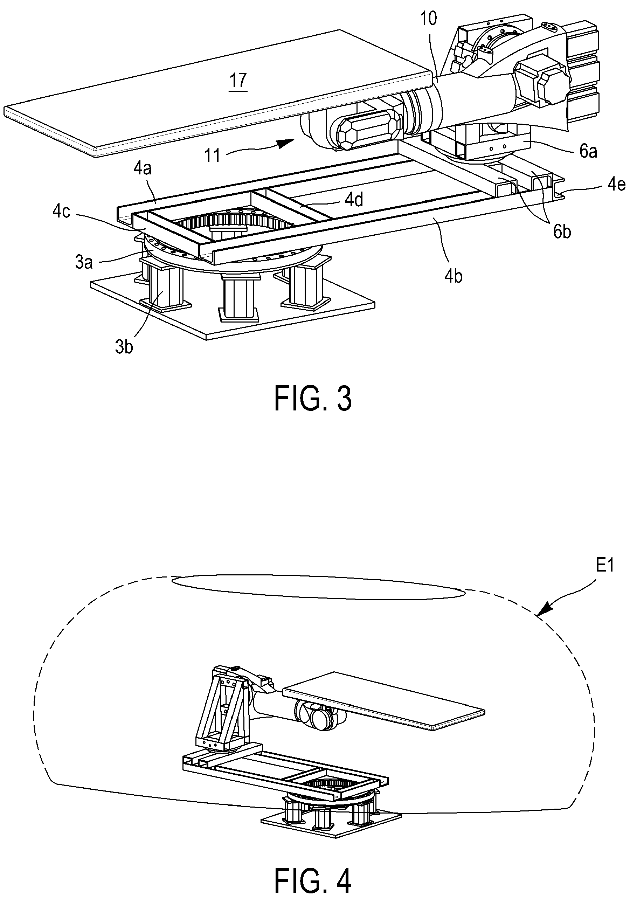

Sur les figures 2 et 3, on voit un exemple de réalisation du robot de positionnement selon l'invention. La base 3 est constituée par une couronne 3a, partie pivotante, montée sur des plots 3b en profilé aluminium solidement fixés au sol.

Le rail linéaire 4 est un cadre formé par deux rails droits, de patins avec roulements 4a et 4b parallèles, de préférence en profilé acier de section en « U ». Ces deux rails 4a et 4b sont reliés entre eux par trois rails 4c, 4d et 4e parallèles entre eux et perpendiculaires avec les deux rails 4a et 4b. Le rail 4c est disposé à une extrémité de l'ensemble rail linéaire. Les deux rails 4c et 4d et une partie des deux rails 4a et 4b constituent un cadre solidement fixé à la couronne 3a de façon pivotante. L'ensemble du rail linéaire 4 peut pivoter en 360° selon l'axe de rotation vertical 5 passant au centre de la couronne 3a.

L'embase 6b est en prise de façon coulissante avec les deux rails 4a et 4b. Cette embase 6b porte de façon pivotante selon l'axe de rotation vertical 7 la pièce de jonction 6a qui est un cadre métallique maintenu verticalement. Le bras robotique 9 est un bras selon l'architecture « SERIE ». Il est fixé à une partie supérieure de la pièce de jonction 6a et peut pivoter selon l'axe de rotation horizontal 12. L'avant bras 10 est un tube allongé et évasé au niveau de la liaison avec la pièce de jonction 6a. L'extrémité libre de l'avant bras porte le poignet 11 sur lequel est fixée la table 17 support pour le patient. Cette table 17 est maintenue sensiblement à l'horizontal la plupart du temps mais elle est amenée à être positionnée par le poignet. La table 17 peut être un plateau ou une chaise dans certains cas pour le transport de patient. Elle est en carbone à base de nid d'abeille afin d'être radio transparente et très rigide tout en gardant un poids limité.

Le robot de positionnement est constitué de telle sorte qu'il est compact en position de repos.

Par exemple, une position de repos peut être la position illustrée sur les figures 2 et 3 dans laquelle le rail linéaire 4 et l'avant bras 10 sont pratiquement parallèles dans un même plan vertical. La pièce de jonction est disposée sur une extrémité du rail linéaire 4 à l'opposé de la base 3. L'avant bras 10 est fixé à la pièce de jonction 6a et est dirigé vers la base 3 tout en restant parallèle au rail linéaire 4. La table 17 est maintenue au-dessus du poignet 11, une extrémité fixée au poignet 11, l'autre extrémité libre étant dirigée du côté opposé à l'avant bras.

Pour améliorer sa compacité, on peut également prévoir un rail linéaire coulissant de sorte qu'en position de repli, l'encombrement soit minimum et/ou le centre de gravité du robot de positionnement soit le plus près possible de l'axe de rotation 5. Avec un rail coulissant, on augmente l'amplitude linéaire de travail, donc l'espace de travail.

La figure 4 est une vue schématique de l'espace de travail E1 pour un robot de positionnement selon l'invention. Cet espace E1 est un volume de forme semi-sphérique aplati sur le dessus. Le patient peut ainsi être positionné de façon continue dans tout le volume de cet espace de travail E1.

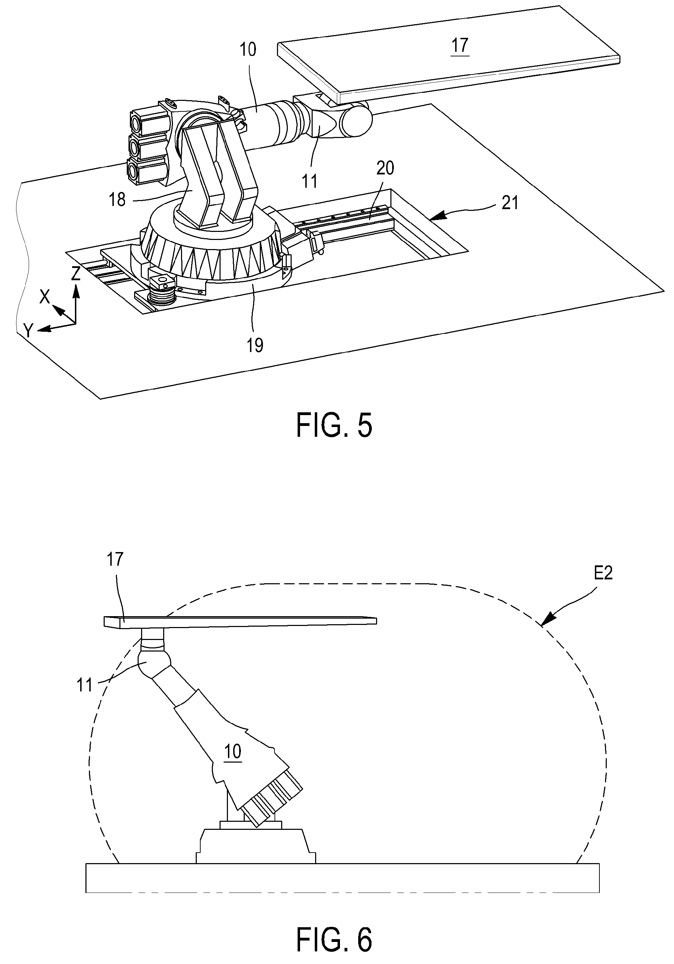

Sur la figure 5, on voit un autre exemple de réalisation dans lequel on retrouve la plupart des composants de l'exemple précédent. La pièce de jonction 18 a été modifiée de sorte que dans ce mode de réalisation, il est possible d'installer une pièce de jonction de grande précision et de poids élevé. En effet, le rail linéaire 21 est fixé au sol et peut recevoir sans peine une pièce de jonction 18 qui peut pivoter sur 360° selon un axe de rotation vertical par rapport à une embase 19. Cette embase 19 coulisse de façon linéaire selon un axe de translation horizontal sur deux rails linéaires 20 parallèles et fixés au sol. Pour la mise en oeuvre de ce mode de réalisation, on prévoit une petite fosse 21 dans laquelle sont installés le premier rail linéaire 20 et le second rail (non visible sur la figure 5). Ce robot de positionnement comporte donc un axe de translation horizontal sur le rail linéaire et cinq axes de rotation : rotation de la pièce de jonction 18 par rapport à l'embase 19, de l'avant bras 10 par rapport à la pièce de jonction, et les trois axes de rotation du poignet 11. On peut également ajouter un autre axe de translation du fait que l'avant bras 10 peut être coulissant.

Avec un tel robot de positionnement, on peut garantir un positionnement précis de la tumeur à **±** 0,5 mm et **±** 0,5 degrés près, ainsi que des possibilités de grands déplacements :
- Delta X = **±** 1000 mm (axe longitudinal en version nominale de l'axe linéaire)
- Delta Y = **±** 1000 mm (axe latéral)
- Delta Z = **±** 800 mm (axe vertical)
- Delta Rx = **±** 20 deg (axe longitudinal)
- Delta Ry = **±** 20 deg (axe latéral)
- Delta Rz = **±** 110 deg (axe vertical)

Sur la figure 6, on voit un exemple de mise en oeuvre dans lequel le robot de positionnement est déployé. Le bras robotique est en position élevée oblique. Le poignet 11 maintient la table 17 en position horizontale. On voit également que l'espace de travail E2 est un volume en forme d'une demi-sphère allongée latéralement et aplati sur le dessus.

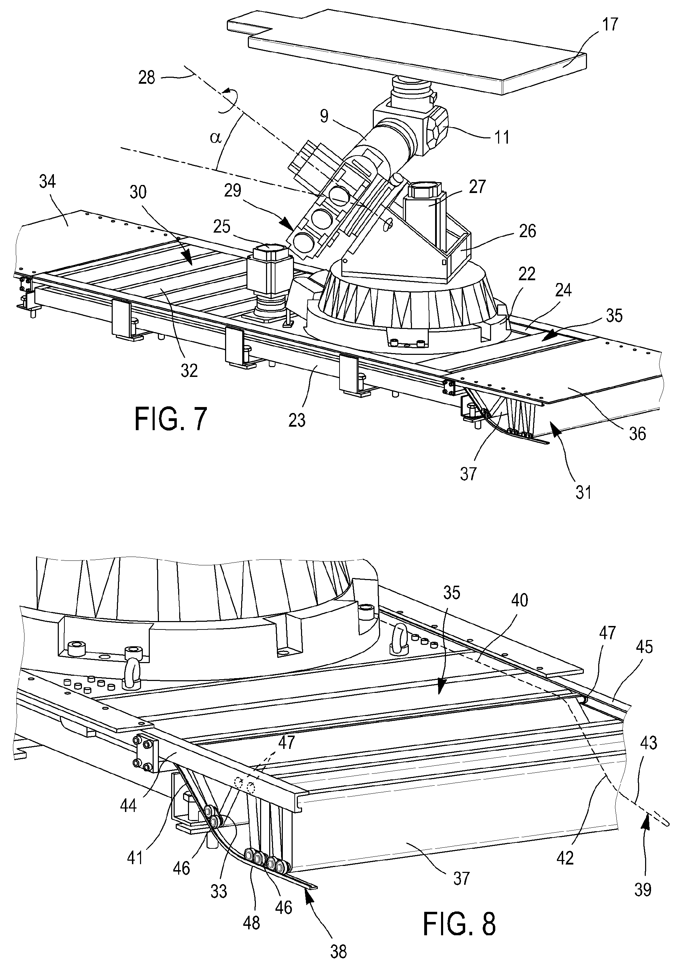

Sur la figure 7 est représenté un autre mode avantageux de réalisation du robot selon l'invention. On distingue une embase 22 qui est apte à coulisser sur deux rails linéaires parallèles 23 et 24 disposés latéralement par rapport à cette embase 22. Un premier moteur 25 participe au déplacement linéaire de l'embase 22. Une pièce de jonction 26, placée au dessus de l'embase 22, est reliée à cette embase 22 de façon pivotante selon un axe vertical. La pièce de jonction présente une forme triangulaire avec la base posée sur l'embase 22 et un flan incliné recevant le bras robotique 9. Un second moteur 27 participe à la rotation de la pièce de jonction 26 par rapport à l'embase 22. Avantageusement, le bras robotique 9 est relié à la pièce de jonction 26 de façon pivotante selon un axe de rotation 28 qui est incliné d'un angle α non nul par rapport à l'horizontal. Cet angle α est de préférence compris entre 45 et 60°. Dans le cas présent, il est de 60°. Cette disposition inclinée permet au robot selon l'invention de se mouvoir dans un espace réduit telle qu'une salle classique d'hôpital avec un plafond limité à 2,5mètres par exemple, et en même temps d'atteindre une hauteur minimale de chargement du patient de l'ordre de 60cm, notamment pour la montée d'un enfant ou d'une personne âgée sur le plateau 17. La disposition inclinée permet de placer le bras robotique 9 et la pièce de jonction 26 sur l'embase 22 le plus proche possible du sol sans que la partie arrière 29 du bras robotique 9 n'entre en collision avec des obstacles, tels que l'embase elle-même, lors de mouvements de rotation. En effet dans les modes de réalisations précédents, le bras robotique 9 est fixé à la pièce de jonction à une hauteur suffisante pour que la rotation du bras robotique n'intercepte pas l'embase 6b par exemple sur la figure 3.

Le robot ainsi décrit possède six axes de manoeuvrabilité, une translation et cinq rotations, soit six degrés de liberté. On peut donc placer un patient dans l'espace de travail dans n'importe quelle configuration.

Selon un autre aspect de l'invention représenté également sur la figure 7, on voit un plancher constitué de deux demi-planchers 30 et 31. Le demi-plancher 30 est composé de plusieurs lames 32 reliées entre elles en chaîne au moyen d'articulations (visible sur la figure 8 en 33). Le demi-plancher 30 est en position dépliée, c'est-à-dire qu'il constitue un plancher plat solide sur lequel peut se tenir l'utilisateur. Ce demi-plancher 30 coulisse en étant solidaire de l'embase 22. Ainsi, lorsque l'embase 22 se déplace en s'éloignant d'un plancher fixe gauche 34, le demi-plancher 30 coulisse en couvrant la fosse qui se trouve entre le plancher fixe gauche 34 et le moteur 25 fixé à l'embase 22. Par contre lorsque l'embase 22 est en buté sur le plancher fixe gauche 34, le demi-plancher 30 coulisse pour se mettre en position de repli, en accordéon, sous le plancher fixe gauche 34. Le demi-plancher 35 agit de la même manière que le demi-plancher 30 mais en opposition de phase. Lorsque l'un est replié, l'autre est déplié, et vice-versa. Sur la figure 7, le demi-plancher 35 est en position de repli sous le plancher fixe droit 36. On voit que les lames 37 constituant le demi-plancher 35 se place en accordéon sous le plancher fixe droit 36.

Sur la figure 8, on voit un peu plus en détail le mécanisme de coulissement du demi-plancher 35, idem pour le demi-plancher 30. Ce demi-plancher 35 coulisse sur deux jeux de rails secondaires.

Un premier jeu de rails secondaires internes 38 et 39 (représenté en pointillé). Chaque rail secondaire 38, 39, comporte une partie linéaire haute 40 (non représentée pour le rail secondaire 38) sur laquelle se trouve les lames 37 en position dépliée permettant à l'utilisateur de marcher dessus ; un point de flexion 41, 42 en forme de « S » et une partie linéaire basse 48, 43 parallèle à la partie linéaire haute 40 mais disposée pratiquement au fond de la fosse.

Un second jeu de rails secondaires externes 44 et 45 présentent un écartement supérieure à celui des deux rails secondaires internes 38, 39. Les deux rails secondaires externes restent linéaires tout le long du déplacement des lames 37. Ces lames portent au niveau des articulations des roulements disposés en quinconce. La moitié des roulements 46 est guidée par les deux rails secondaires internes 38, 39, alors que l'autre moitié 47 est guidée par les deux rails secondaires externes 44 et 45, voir figure 8. Plus précisément, ces roulements sont disposés de façon alternative, un roulement 46 est suivi d'un roulement 47 et vice versa. En position de repli, les roulements 47 coulissent dans les rails secondaires externes 44 et 45, alors que les roulements 46 coulissent dans le point de flexion 41, 42 qui constitue une rampe d'accès vers un niveau inférieur où se trouve la partie linéaire basse 43, 48. Ainsi, en position de repli, les lames 37 sont disposées à la verticale et en accordéon. Elles occupent alors un volume minimum et restent camouflées. Les lames 37 passent de l'état horizontal à l'état vertical juste avec l'effet de la gravité, elles se rangent automatiquement et sans motorisation supplémentaire.

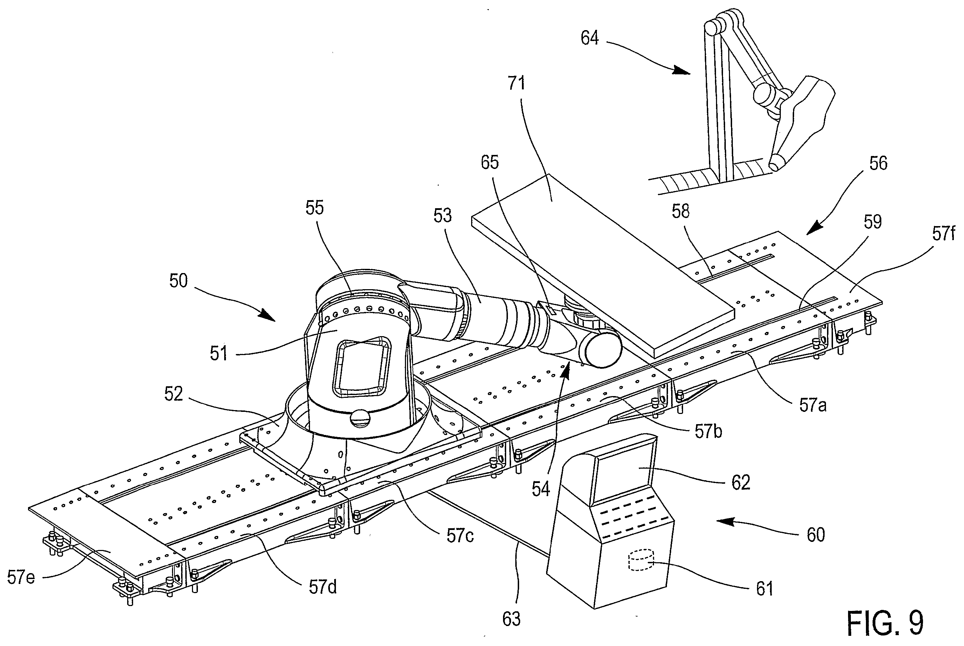

Sur la figure 9, on voit un mode de réalisation du système selon l'invention dans une salle de traitement équipée du dispositif de positionnement selon l'invention ainsi que d'éléments fixes et mobiles.

Le dispositif de positionnement peut être tel que décrit précédemment. De préférence, on utilise le dispositif de positionnement de la figure 9 comprenant une pièce de jonction 50 coulissant sur un rail linéaire 56 et portant un bras robotique 53 doté d'un poignet 54 à axes de rotation concourants.

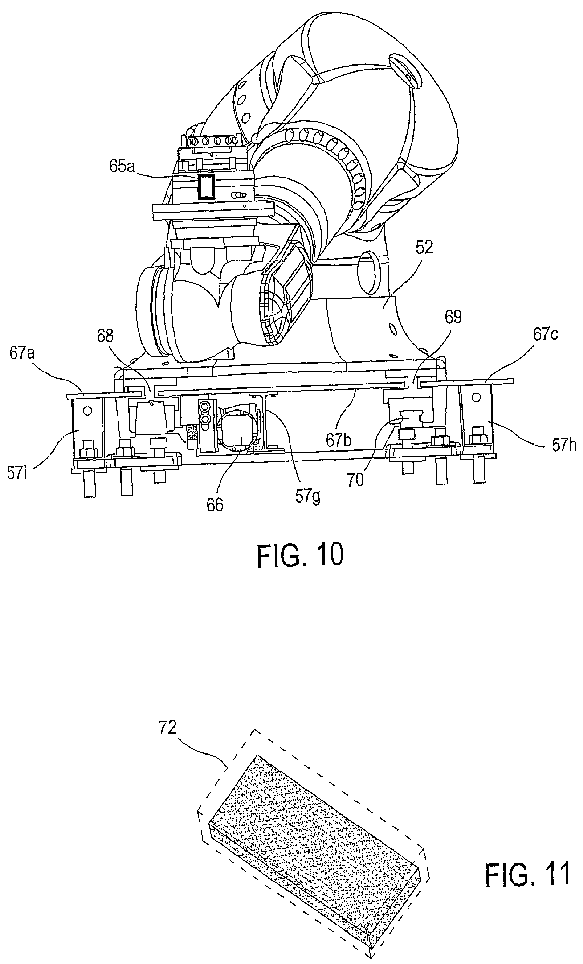

Le rail linéaire 56 est avantageusement fixés au sol et est constitué de plusieurs éléments modulaires 57a,..., 57d reliés les uns aux autres. Ces éléments modulaires peuvent être identiques de sorte que leur mise en place dans la salle de traitement soit facilitée. Avec un tel arrangement, il est ainsi aisé de réaliser des rails linéaires de longueurs différentes. Comme on le voit sur la figure 10 qui représente une vue de côté du dispositif de positionnement de la figure 9 sans le support de patient, chaque élément modulaire 57a,..., 57d, comporte une plaque métallique (ou autre matériau solide tel que le bois, plastique,...) supérieure 67a, 67b, 67c posée sur trois montants solidement fixées au sol, deux montants latéraux 57h, 57i, et un montant central 57g. Chaque plaque métallique supérieure comporte deux rainures ou ouvertures latérales 58 et 59 sur la figure 9 qui sont parallèles et subdivisent chaque plaque métallique supérieure en trois parties 67a, 67b, 67c. Ces rainures donnent accès à un volume de confinement entre les plaques métalliques supérieures 67b et le sol. Ce volume de confinement renferme un moteur 66 sur la figure 10 destiné à faire coulisser l'embase 52 de la pièce de jonction 50 par rapport au rail linéaire 56. Plus précisément, l'embase 52 est portée par des patins 68 et 69. Ces patins comprennent une partie supérieure pour supporter l'embase 52, une partie inférieure coulissant sur des rails fixes 70 dans le volume de confinement, et une partie centrale conformée de façon suffisamment étroite et solide pour relier la partie supérieure et la partie inférieure via les rainures 58 et 59 sans jamais les toucher.

Une telle réalisation permet de camoufler le moteur dans un volume de confinement invisible de l'extérieur, cela permet de gagner de la place et rend la surface supérieure du rail linéaire plane. L'utilisateur peut se déplacer en toute sécurité sur ce plancher constitué par les plaques métalliques supérieures des éléments modulaires 57a,..., 57d. On prévoit également deux éléments modulaires de butée 57e et 57f qui sont respectivement disposés aux deux extrémités du rail linéaire 56.

L'embase 52 est associée à une pièce de pivot 51 apte à pivoter selon un axe de rotation vertical. Le bras robotique 53 est relié de façon pivotante à une partie supérieure de la pièce de jonction 50 selon un axe de rotation faisant un angle compris entre 45 et 60° par rapport à l'horizontal. Le poignet 54 porte un support de patient 71 qui peut être positionné de manière très précise dans le référentiel de la salle de traitement.

Selon l'invention, une unité de traitement 60 permet de piloter de façon électromécanique le dispositif de positionnement ou robot. Plusieurs moteurs dont le moteur 66 pour le déplacement du rail linéaire sont disposés sur et dans le robot de façon à contrôler toute articulation du robot de façon automatique. Un ensemble de capteurs conventionnels sont disposés sur le robot tel que par exemple un inclinomètre 65 disposé sur le poignet 54. A partir des capteurs ainsi que notamment des moteurs, l'unité de traitement récupère un ensemble d'information permettant de connaître exactement en temps réel le positionnement du robot. C'est-à-dire qu'à chaque instant on connaît la position du support dans la salle et la valeur des angles d'inclinaison des différents éléments du robot.

L'unité de traitement comprend une partie matérielle de type ordinateur doté d'éléments conventionnels pour l'acquisition, le traitement analogique et numérique de données. Un disque dur 61 loge un module de visualisation 3D qui détermine puis affiche sur un écran 62 une représentation 3D du déplacement du robot par rapport à l'environnement qui est la salle de traitement. Ce module de visualisation comporte donc une modélisation 3D virtuelle de l'environnement et une modélisation 3D virtuelle temps réel du robot se déplaçant dans l'environnement. Avantageusement, il comporte également une modélisation 3D virtuelle d'une enveloppe virtuelle autour du support 71 du robot ainsi qu'un algorithme de détection de collisions temps réel entre l'enveloppe virtuelle et l'environnement modélisé.

La modélisation de l'environnement tient compte des dimensions de la salle de traitement mais également des éléments ou obstacles présents dans cette salle de traitement. On distingue notamment l'unité de traitement elle-même ainsi qu'un dispositif de rayonnement 64 qui est généralement mobile. Avantageusement, l'unité de traitement est reliée au robot et au dispositif de rayonnement 64 de façon filaire 63 ou sans fil, de sorte que le module de visualisation 3D peut représenter tout appareil mobile dans la salle de traitement.

Les modélisations sont obtenues à partir de données acquises en temps réel et de données prédéterminées. Ces données prédéterminées peuvent correspondre aux données de positionnement des éléments mobiles. Ces positions, comme celle de l'unité de traitement, sont connues d'avance et peuvent être saisies par l'utilisateur.

La représentation virtuelle de la dynamique dans la salle de traitement permet de mettre en place des systèmes de surveillance telle qu'un processus anticollision.

Pour ce faire, on prévoit que l'enveloppe virtuelle suive le mouvement du support 71. La figure 11 est une représentation 3D virtuelle visible sur l'écran 62. Seul le support 71 est représenté pour des raisons de simplification. L'enveloppe virtuelle 72 est de la même forme que la représentation virtuelle du support 71 mais de dimensions supérieures. Par conséquent, lorsque le support 71 est en mouvement, l'enveloppe 72 suit le même mouvement et toute collision probable du support 71 avec un des éléments de la salle de traitement est précédée par une collision virtuelle de l'enveloppe 72 dans le module de visualisation 3D. En fait, la représentation 3D permet d'alerter l'utilisateur du risque de collision réel du support 71 lorsque l'enveloppe virtuelle 72 entre en collision virtuelle.

Sur la figure 11 l'enveloppe 72 englobe la représentation 3D du support 71, mais cette enveloppe 72 peut être de forme différente de celle du support et de taille inférieure, notamment pour ne surveiller qu'une partie du support.

La modélisation 3D d'une enveloppe virtuelle peut également être utilisée sur tout élément mobile de la salle de traitement. On peut ainsi prévoir une seconde enveloppe virtuelle autour du dispositif de rayonnement 64, la collision étant estimée entre les deux enveloppes virtuelles.

En pratique, le module de visualisation peut être mis en oeuvre à partir notamment d'un moteur logiciel 3D et de techniques provenant du monde des jeux vidéo (moteur physique) pour le calcul des collisions de manière optimale. La détection de collisions est issue de puissants algorithmes optimisés tels que notamment les algorithmes suivants connus de l'homme du métier :
- algorithme de type « n-body pruning »,
- algorithme de la cohérence temporelle,
- algorithme de distance de type Gilbert-Johnson-Keerthi,

Ces algorithmes permettent d'augmenter la vitesse de détection de collision. On peut envisager environ 60 tests de collision par seconde.

Lorsqu'une collision est détectée, il existe plusieurs possibilités d'action. On peut demander l'arrêt complet du système ou réorienter le mouvement du robot pour éviter la collision réelle (par exemple glissement du robot sur une surface virtuelle).

Un tel système anticollision présente de nombreux avantages:
- Sécurisation et protection des personnes circulant dans la salle de traitement.
- Système de protection extérieur à la chaine de fonctionnement normale pour le matériel concerné.
- Augmentation des possibilités de mouvement, les éléments mobiles étant protégés de la collision, et donc augmentation du confort des opérateurs quand à la manoeuvrabilité de ces éléments.

Un système anticollision permet d'augmenter la capacité de l'usage d'un système mobile dans l'espace. Un robot médicale par exemple, est ainsi manoeuvrable facilement par l'opérateur en toute sécurité sans qu'il n'ait besoin de se soucier d'un éventuel contact. Les machines sont donc plus autonomes, elles assurent elles mêmes leur propre sécurité et celle de leur entourage.

Selon l'invention, on prévoit d'améliorer la manipulation du robot par un processus de co-manipulation qui consiste à détecter une force appliquée au robot puis à commander électro-mécaniquement le robot de façon à favoriser le mouvement induite par cette force. La force appliquée provient généralement d'un utilisateur qui déplace manuellement le robot en poussant par exemple le support de patient à l'aide de ses mains.

Le processus de co-manipulation peut être un processus indépendant ou bien associé à la technique anticollision avec enveloppe virtuelle. Dans ce cas, lors du déplacement manuel du robot lors d'une co-manipulation par exemple, l'unité de traitement met en oeuvre en même temps la détection de collision.

Sur la figure 8 par exemple, le capteur 65a peut être un capteur d'effort utilisé pour détecter une quelconque force appliquée sur le poignet 54. Ce type de capteur d'effort peut être constitué de plusieurs jauges de contraintes. On peut prévoir plusieurs capteurs d'effort répartis sur plusieurs éléments du robot de façon à appréhender toute force appliquée sur ce robot.

Lorsqu'on décide de réorienter le mouvement du robot, le processus anticollision permet un glissement lent du support dans le volume de l'enveloppe virtuelle. Ce principe permet en mode co-manipulation d'éviter des arrêts intempestifs du robot et de lisser les trajectoires aux abords d'éléments présents dans la salle de traitement. Le glissement est en outre une assistance à la manipulation et au guidage manuel du robot.

Bien sûr, l'invention n'est pas limitée aux exemples qui viennent d'être décrits et de nombreux aménagements peuvent être apportés à ces exemples sans sortir du cadre de l'invention. On peut notamment prévoir un seul rail linéaire central sur lequel l'embase peut coulisser.

## Revendications

1. Dispositif pour positionner un patient par rapport à un rayonnement (2), ce dispositif comprenant :
- au moins un rail linéaire (23, 24) de déplacement horizontal,
- une pièce de jonction (26) apte à réaliser des translations par rapport au rail linéaire et à pivoter selon un axe de rotation vertical par rapport à ce rail linéaire, et qui est reliée de façon pivotante selon un axe vertical à une embase (6b) qui est reliée de façon coulissante audit au moins un rail linéaire (23, 24),
- un bras robotique (9) relié à la pièce de jonction (26), ce bras robotique comprenant un poignet (11) à axes de rotations (14, 15, 16) concourants relié à un support (17) de patient,
**caractérisé en ce que** le bras robotique (9) est relié à la pièce de jonction (26) de façon pivotante selon un axe de rotation incliné par rapport à l'horizontal d'un angle α non nul compris entre 0° et 90°.

2. Dispositif selon la revendication 1, **caractérisé en ce que** l'axe de rotation du bras robotique (9) par rapport à la pièce de jonction (26) est incliné par rapport à l'horizontal d'un angle α compris entre 45 et 60 degrés.

3. Dispositif selon la revendication 1 ou 2, **caractérisé en ce que** le rail linéaire (23, 24) est fixé au sol.

4. Dispositif selon l'une quelconque des revendications 1 à 3, **caractérisé en ce que** le rail linéaire est fixé à une partie pivotante (3a) d'une base (3), l'axe de pivot de cette partie pivotante (3a) étant vertical (5).

5. Dispositif selon la revendication 4, **caractérisé en ce que** l'axe de rotation vertical (5) du rail linéaire (23, 24) par rapport à la base (3) est confondu avec l'axe dudit rayonnement (2).

6. Dispositif selon l'une quelconque des revendications précédentes, **caractérisé en ce que** le bras robotique (9) comprend un avant bras (10) coulissant.

7. Dispositif selon l'une quelconque des revendications précédentes, **caractérisé en ce que** le poignet (11) est relié au support (17) de patient au moyen d'un changeur d'outil électropneumatique industriel.

8. Dispositif selon l'une quelconque des revendications précédentes, **caractérisé en ce qu'**il comprend des roues (23) amovibles qui portent le dispositif lors d'une installation.

9. Dispositif selon l'une quelconque des revendications précédentes, **caractérisé en ce qu'**il comporte un plancher mobile constitué de deux demi-planchers (30, 35) reliés à la pièce de jonction (26) et disposés de part et d'autre de cette pièce de jonction (6a) de façon à couvrir constamment ledit au moins un rail linéaire (4) lorsque la pièce de jonction se déplace ;
chaque demi-plancher (30, 35) est constitué de plusieurs lames (32, 37) reliées entre elles en chaîne par des articulations (33), de telle sorte qu'en position de repli d'un demi-plancher, au moins une partie des lames de ce demi-plancher est rangée en accordéon sous un plan horizontal ; ce plan horizontal étant tel que ce demi-plancher, en position dépliée, est contenu dans ce plan horizontal.

10. Dispositif selon la revendication 9, **caractérisé en ce que** le plancher coulisse sur deux jeux de rails secondaires :
- un premier jeu de rails secondaires internes (38, 39) sur lesquels coulisse la moitié (46) des articulations, ces deux rails secondaires internes (38, 39) étant parallèles et écartés l'un de l'autre ; ces deux rails secondaires internes présentant une partie linéaire haute (40), un point de flexion (41, 42) et une partie linéaire basse (43, 48) ;
- un second jeu constitué de deux rails secondaires externes (44, 45) sur lesquels coulisse l'autre moitié (47) des articulations, ces deux rails secondaires externes (44, 45) étant parallèles et écartés d'une distance supérieure à la distance d'écartement des deux rails secondaires internes (38, 39) ; ces deux rails secondaires externes étant linéaires dans un plan horizontal sensiblement à la même hauteur que la partie linéaire haute des rails secondaires internes ;
et **en ce que** pour deux articulations successives, l'une est portée par le premier jeu de rails secondaires, l'autre étant portée par le second jeu de rails secondaires.

11. Dispositif selon la revendication 10, **caractérisé en ce que** chaque demi-plancher présente en position dépliée une partie plate proche de la pièce de jonction, et en position de repli une partie repliée en accordéon entre la partie linéaire basse des rails secondaires internes et les rails secondaires externes.

12. Dispositif selon l'une quelconque des revendications 1 à 8, **caractérisé en ce que** le rail linéaire (56) est constitué par une pluralité d'éléments modulaires (57a,..., 57d) fixés au sol et connectés entre eux.

13. Dispositif selon l'une quelconque des revendications précédentes, **caractérisé en ce qu'**il comprend au moins un capteur d'effort (65a) fixé au poignet (54) et relié à une unité de traitement (60) contrôlant ledit dispositif de positionnement (50, 53, 54, 71) de façon à réaliser une co-manipulation en accompagnant tout effort détecté par ledit au moins un capteur d'effort.

14. Système de positionnement comprenant :
- une unité de traitement intégrant un logiciel de supervision,
- des capteurs de recalage d'un patient,
- un dispositif pour positionner le patient par rapport à un rayonnement (2) selon l'une quelconque des revendications précédentes.

15. Système selon la revendication 14, **caractérisé en ce que** les capteurs comprennent l'un au moins des capteurs suivants : un capteur stéréovision infrarouge, des capteurs rayons X, des caméras industrielles, et un capteur d'effort.

16. Système selon la revendication 14 ou 15, **caractérisé en ce que** les capteurs comprennent l'un au moins des capteurs suivants : résolveur, accéléromètre, capteur de survitesse, inclinomètre, et bande anti collision.

17. Système selon l'une des revendications 14 à 16, **caractérisé en ce que** l'unité de traitement (60) comprend un module de visualisation 3D doté :
- d'une modélisation 3D virtuelle de la salle de traitement où se situe le dispositif de positionnement ;
- d'une modélisation 3D virtuelle du dispositif de positionnement (50, 53, 54, 71) ; cette modélisation étant paramétrée pour représenter le positionnement temps réel du dispositif de positionnement à partir de capteurs, et pour définir une enveloppe virtuelle (72) autour au moins d'un élément du dispositif de positionnement de façon à générer un signal d'alerte lorsque l'enveloppe virtuelle entre en collision avec une représentation virtuelle d'un élément de la salle de traitement.

18. Système selon la revendication 17, **caractérisé en ce que** l'unité de traitement (60) est paramétrée de façon à déclencher, en réponse au signal d'alerte, un processus anticollision consistant à arrêter le dispositif de positionnement ou à réorienter le mouvement du dispositif de positionnement.

## Patentansprüche

1. Vorrichtung zur Positionierung eines Patienten gegenüber einer Strahlenquelle (2), umfassend:
- wenigstens eine Linearschiene (23, 24) mit horizontaler Bewegung,
- ein Verbindungsstück (26), das dazu geeignet ist, Verstellungen gegenüber der Linearschiene durchzuführen und um eine vertikale Drehachse gegenüber der Linearschiene zu schwenken, und das schwenkbar um eine vertikale Achse an einem Sockel (6b) verbunden ist, welcher gleitend mit der mindestens einen Linearschiene (23, 24) verbunden ist,
- einen mit dem Verbindungsstück (26) verbundenen Roboterarm (9), wobei dieser Roboterarm ein Handgelenk (11) mit zusammenlaufenden Drehachsen (14, 15, 16) umfasst, das mit einem Patiententräger (17) verbunden ist,
**dadurch gekennzeichnet, dass** der Roboterarm (9) mit dem Verbindungsstück (26) drehbar um eine Drehachse verbunden ist, die mit einem Neigungswinkel α nicht gleich Null zwischen 0° und 90° zur Horizontale gelagert ist.

2. Vorrichtung nach Anspruch 1, **dadurch gekennzeichnet, dass** die Drehachse des Roboterarms (9) gegenüber dem Verbindungsstück (26) mit einem Neigungswinkel α zwischen 45 und 60° zur Horizontale gelagert ist.

3. Vorrichtung nach Anspruch 1 oder 2, **dadurch gekennzeichnet, dass** die Linearschiene (23, 24) am Fußboden befestigt ist.

4. Vorrichtung nach einem der vorhergehenden Ansprüche 1-3, **dadurch gekennzeichnet, dass** die Linearschiene an einem schwenkbaren Teil (3a) eines Fundaments (3) befestigt ist, wobei die Schwenkachse dieses schwenkbaren Teils (3a) vertikal (5) gelagert ist.

5. Vorrichtung nach Anspruch 4, **dadurch gekennzeichnet, dass** die vertikale Drehachse (5) der Linearschiene (23, 24) gegenüber des Fundaments (3) ähnlich der Achse der Strahlenquelle (2) verläuft.

6. Vorrichtung nach einem der vorhergehenden Ansprüche, **dadurch gekennzeichnet, dass** der Roboterarm (9) einen gleitenden Vorderarm (10) umfasst.

7. Vorrichtung nach einem der vorhergehenden Ansprüche, **dadurch gekennzeichnet, dass** das Handgelenk (11) mittels eines industriellen elektropneumatischen Werkzeugwechslers mit dem Patiententräger (17) verbunden ist.

8. Vorrichtung nach einem der vorhergehenden Ansprüche, **dadurch gekennzeichnet, dass** sie abnehmbare Räder (23) umfasst, welche die Vorrichtung bei einer Installation tragen.

9. Vorrichtung nach einem der vorhergehenden Ansprüche, **dadurch gekennzeichnet, dass** sie einen fahrbaren Boden aus zwei, mit dem Verbindungsstück (26) verbundenen Halbböden (30, 35) umfasst, welche an beiden Seiten des Verbindungsstücks (6a) derart angeordnet sind, dass sie die mindestens eine Linearschiene (4) stets zudecken, wenn das Verbindungsstück sich bewegt;
jeder Halbboden (30, 35) besteht aus mehreren, miteinander kettenweise durch Gelenke (33) verbundenen Platten (32, 37), sodass wenn ein Halbboden zurückgeklappt ist, mindestens ein Teil der Platten dieses Halbbodens harmonikaförmig unter einer horizontalen Ebene angeordnet ist; wobei diese horizontale Ebene so ausgebildet ist, dass der Halbboden in aufgefalteter Position in der horizontalen Ebene hinein passt.

10. Vorrichtung nach Anspruch 9, **dadurch gekennzeichnet, dass** der Boden auf zwei Sätzen Nebenschienen gleitet:
- ein erster Satz Innennebenschienen (38, 39), auf denen die Hälfte (46) der Gelenke gleitet, wobei diese beiden Innennebenschienen (38,39) parallel zueinander und voneinander beabstandet gelagert sind; wobei diese beiden Innennebenschienen einen oberen Linearabschnitt (40), einen Biegepunkt (41, 42) und einen unteren Linearabschnitt (43, 48) auf weisen,
- einen zweiten Satz, bestehend aus zwei Außennebenschienen (44, 45), auf denen die andere Hälfte (47) der Gelenke gleitet, wobei die Außennebenschienen (44, 45) parallel zueinander und in einem größeren Abstand als der Abstand zwischen den beiden Innennebenschienen (38,39) voneinander beabstandet sind; wobei die Außennebenschienen linear in einer horizontalen Ebene, im Wesentlichen an der gleichen Höhe wie der obere Linearabschnitt der Innennebenschienen verlaufen;
und dadurch, dass für zwei aufeinander folgende Gelenke die eine von dem ersten Satz Nebenschienen, die andere von dem zweiten Satz Nebenschienen getragen wird.

11. Vorrichtung nach Anspruch 10, **dadurch gekennzeichnet, dass** jeder Halbboden in aufgefalteter Position einen flachen, dem Verbindungsstück nah angeordneten Teil und in gefalteter Position einen harmonikaförmig gefalteten Teil zwischen dem unteren Linearabschnitt der Innennebenschienen und den Außennebenschienen aufweist.

12. Vorrichtung nach einem der Ansprüche 1-8, **dadurch gekennzeichnet, dass** die Linearschiene (56) aus einer Vielzahl von Modulelementen (57a, ..., 57d) besteht, die am Fußboden befestigt und miteinander verbunden sind.

13. Vorrichtung nach einem der vorhergehenden Ansprüche, **dadurch gekennzeichnet, dass** sie mindestens einen Kraftsensor (65a) umfasst, der am Handgelenk (54) befestigt und an einer Behandlungseinheit (60) angeschlossen ist, welche die Positionierungsvorrichtung (50, 53, 54, 71) so steuert, dass eine Mitbedienung ausgeführt wird, wobei jede durch den mindestens einen Kraftsensor erkannte Kraft begleitet wird.

14. System zur Positionierung, umfassend:
- eine Behandlungseinheit mit integrierter Überwachungssoftware,
- Sensoren zur Neuaufrichtung eines Patienten,
- eine Vorrichtung zur Positionierung des Patienten gegenüber einer Strahlenquelle (2) nach einem der vorhergehenden Ansprüche.

15. System nach Anspruch 14, **dadurch gekennzeichnet, dass** die Sensoren mindestens einen der folgenden Sensoren umfassen: einen optischen Infrarot-Stereosensor, Sensoren für Röntgenstrahlen, industrielle Kameras, sowie einen Kraftsensor.

16. System nach Anspruch 14 oder 15, **dadurch gekennzeichnet, dass** die Sensoren mindestens einen der folgenden Sensoren umfassen: Koordinatenwandler, Beschleunigungsmesser, Überdrehzahlsensor, Gefällmesser sowie Antikollisionsband.

17. System nach einem der Ansprüche 14-16, **dadurch gekennzeichnet, dass** die Behandlungseinheit (60) ein Modul zur 3D-Sichtanzeige umfasst mit:
- einer virtuellen 3D-Modellierung des Behandlungsraums, in dem die Positionierungsvorrichtung steht;
- einer virtuellen 3D-Modellierung der Positionierungsvorrichtung (50, 53, 54, 71); wobei die Modellierung so eingestellt ist, dass die Echtzeit-Positionierung der Positionierungsvorrichtung anhand von Sensoren dargestellt wird und dass eine virtuelle Hülle (72) um mindestens einen Bestandteil der Positionierungsvorrichtung bestimmt wird, sodass ein Alarmsignal ausgegeben wird, wenn die virtuelle Hülle mit einer virtuellen Darstellung eines Bestandteils des Behandlungsraums zusammenstößt.

18. System nach Anspruch 17, **dadurch gekennzeichnet, dass** die Behandlungseinheit (60) so eingestellt ist, dass als Antwort auf das Alarmsignal ein Antikollisionsprozess gestartet wird, welcher darin besteht, die Positionierungsvorrichtung zu stoppen oder die Bewegung der Positionierungsvorrichtung neu auszurichten.

## Claims

1. Device for positioning a patient with respect to a radiation (2), this device comprising:
- at least one horizontal displacement linear rail (23, 24),
- a connecting part (26) capable of carrying out translations with respect to the linear rail and of pivoting about an axis of rotation vertical with respect to that linear rail, and said connecting part (26) is connected in a pivoting manner about an axis vertical to a seat (6b) which is connected in a sliding manner to said at least one linear rail (23, 24),
- a robotic arm (9) connected to the connecting part (26), this robotic arm comprising a wrist (11) with concurrent axes of rotation (14, 15, 16) connected to a patient support (17),
**characterized in that** the robotic arm (9) is connected to the connecting part (26) in a pivoting manner about an axis of rotation which is inclined with respect to the horizontal by a non-zero angle of between 0° and 90°.

2. Device according to claim 1, **characterized in that** the axis of rotation of the robotic arm (9) with respect to the connecting part (26) is inclined with respect to the horizontal by an angle of between 45 and 60 degrees.

3. Device according to claim 1 or 2, **characterized in that** the linear rail (4) is fixed to the ground.

4. Device according to any one of claims 1 to 4, **characterized in that** the linear rail is fixed to a pivoting part (3a) of a base (3), the pivot axis of this pivoting part (3a) being vertical (5).

5. Device according to claim 6, **characterized in that** the vertical axis of rotation (5) of the linear rail (23, 24) with respect to the base (3) is merged with the axis of said radiation (2).

6. Device according to any one of the preceding claims, **characterized in that** the robotic arm (9) comprises a sliding forearm (10).

7. Device according to any one of the preceding claims, **characterized in that** the wrist (11) is connected to the patient support (17) by means of an industrial electro-pneumatic tool changer.

8. Device according to any one of the preceding claims, **characterized in that** it comprises removable wheels (23) which support the device during installation.

9. Device according to any one of the preceding claims, **characterized in that** it comprises a mobile platform constituted by two half-platforms (30, 35) connected to the connecting part (26) and arranged on each side of this connecting part (6a) in order to constantly cover said at least one linear rail (4) when the connecting part moves;
each half-platform (30, 35) is constituted by several slats (32, 37) chainlinked to each other by hinges (33), such that when a half-platform is in the folded position, at least some of the slats of this half-platform are concertinaed under a horizontal plane; this horizontal plane being such that this half-platform, in the unfolded position, is contained in this horizontal plane.

10. Device according to claim 9, **characterized in that** the platform slides on two sets of secondary rails:
- a first set of inner secondary rails (38, 39) upon which slides half (46) of the hinges, these two inner secondary rails (38, 39) being parallel and separated from each other; these two inner secondary rails having an upper linear part (40), a bend point (41, 42) and a lower linear part (43, 48);
- a second set constituted by two outer secondary rails (44, 45) upon which slides the other half (47) of the hinges, these two outer secondary rails (44, 45) being parallel and separated by a distance greater than the distance separating the two inner secondary rails (38, 39); these two outer secondary rails being linear in a horizontal plane substantially at the same height as the upper linear part of the inner secondary rails;
and **in that** for two successive hinges, one is borne by the first set of secondary rails, the other being borne by the second set of secondary rails.

11. Device according to claim 10, **characterized in that** each half-platform has, in the unfolded position, a flat part close to the connecting part and, in the folded position, a part concertinaed between the lower linear part of the inner secondary rails and the outer secondary rails.

12. Device according to any one of claims 1 to 8, **characterized in that** the linear rail (56) is constituted by a plurality of modular elements (57a,..., 57d) fixed to the floor and connected to each other.

13. Device according to any one of the preceding claims, **characterized in that** it comprises at least one force sensor (65a) fixed to the wrist (54) and connected to a processing unit (60) controlling said positioning device (50, 53, 54, 71) in such a way as to carry out a co-manipulation accompanying any force detected by said at least one force sensor.

14. Positioning system comprising:
- a processing unit integrating supervision software,
- patient resetting sensors,
- a device for positioning a patient with respect to a radiation (2) according to any one of the preceding claims.

15. System according to claim 14, **characterized in that** the sensors include at least one of the following sensors: an infrared stereovision sensor, X-ray sensors, industrial cameras and a force sensor.

16. System according to claim 14 or 15, **characterized in that** the sensors include at least one of the following sensors: resolver, accelerometer, overspeed sensor, inclinometer, and anti-collision strip.

17. System according to one of claims 14 to 16, **characterized in that** the processing unit (60) comprises a 3D display module provided with:
- a virtual 3D modelling of the treatment room in which the positioning device is located;
- a virtual 3D modelling of the positioning device (50, 53, 54, 71); this modelling being parameterized in order to represent the real-time positioning of the positioning device on the basis of sensors, and to define a virtual envelope (72) around at least one element of the positioning device in order to generate an alarm signal when the virtual envelope collides with a virtual representation of an element of the treatment room.

18. System according to claim 17, **characterized in that** the processing unit (60) is parameterized in order to initiate, in response to the alarm signal, an anti-collision process consisting in the stopping of the positioning device or the reorientation of the movement of the positioning device.
